# EUROPÄISCHE PATENTSCHRIFT

(11) **EP 3 013 478 B1**
(45) Veröffentlichungstag und Bekanntmachung des Hinweises auf die Patenterteilung: **29.07.2020**
(21) Anmeldenummer: 14746947.2
(22) Anmeldetag: 11.06.2014
(51) Int. Cl.: A61B 10/00, B01L 3/00

(54) **VORRICHTUNG UND VERFAHREN ZUR AUFBEWAHRUNG UND ZUM TRANSPORT EINER KÖRPERFLÜSSIGKEITSPROBE**
DEVICE AND METHOD FOR STORAGE AND TRANSPORT OF BODY FLUID SAMPLE
DISPOSITIF ET PROCÉDÉ DE STOCKAGE ET DE TRANSPORT D'UN ÉCHANTILLON DE FLUIDE CORPOREL

(30) Priorität: 24.06.2013 DE 102013211918
(43) Veröffentlichungstag der Anmeldung: 04.05.2016
(73) Patentinhaber: Euroimmun Medizinische Labordiagnostika AG, 23560 Lübeck (DE)
(72) Erfinder: HERBST, Victor, 23628 Krummesse (DE); WICHNER, Birgit, 23566 Lübeck (DE)
(86) Internationale Anmeldenummer: PCT/EP2014/001583
(87) Internationale Veröffentlichungsnummer: WO 2014/206531

(56) Entgegenhaltungen:
- EP-A1- 2 420 831
- WO-A1-00/54029
- WO-A1-2006/034592
- US-A- 3 162 306
- US-A- 3 996 006
- US-A- 5 683 915
- US-A- 5 939 259
- US-A1- 2013 143 226
- FUMIN LI ET AL: "Perforated dried blood spots: a novel format for accurate microsampling", BIOANALYSIS, Bd. 3, Nr. 20, 1. Oktober 2011 (2011-10-01), Seiten 2321-2333, XP055147130, ISSN: 1757-6180, DOI: 10.4155/bio.11.219
- FUMIN LI ET AL: "Perforated dried blood spot accurate microsampling: the concept and its applications in toxicokinetic sample collection", JOURNAL OF MASS SPECTROMETRY, Bd. 47, Nr. 5, 10. Mai 2012 (2012-05-10), Seiten 655-667, XP055147255, ISSN: 1076-5174, DOI: 10.1002/jms.3015

## Beschreibung

Die Erfindung betrifft eine Vorrichtung sowie ein Verfahren zur Aufbewahrung und zum Transport einer Körperflüssigkeitsprobe von einem Ort der Probenahme zum Labor, optional weiterhin zur Prozessierung der Probe. Die Vorrichtung weist als wesentliche Komponenten ein Tragelement als Grundkörper, wenigstens ein Sorptionselement zur Aufnahme einer flüssigen Probe sowie ein an das Sorptionselement angrenzendes Überlaufreservoir auf. Das Sorptionselement verfügt über ein definiertes Füllvolumen zur Aufnahme eines Tropfens einer zu untersuchenden Körperflüssigkeit. Sobald das Sorptionselement vollständig mit Probenflüssigkeit gefüllt ist wird überschüssige Flüssigkeit von dem angrenzenden Überlaufreservoir aufgenommen.

Auf dem Gebiet der In-vitro-Diagnostik sind allgemein Untersuchungen bekannt, bei denen einem Patienten ein Tropfen Blut entnommen wird, der daraufhin auf ein hierfür vorgesehenes Filterpapierfeld einer Testkarte aufgetropft und mit der Karte in ein Labor eingesendet wird. Um im Labor die Blutprobe des Patienten auf geeignete Weise untersuchen zu können, werden die mit dem Blut versehenen Felder üblicherweise zunächst mit Hilfe einer Stanze aus der Karte ausgestanzt.

Die zuvor erwähnten Untersuchungen werden vornehmlich zum Screening, insbesondere zum Screening von Neugeborenen, eingesetzt. In diesem Zusammenhang ist vor allem der in den frühen 1960er Jahren von Robert Guthrie entwickelte Guthrie-Test bekannt. Bei diesem Test wird aus der Ferse des Neugeborenen Blut auf hierfür vorgesehene Felder einer Filterpapierkarte getropft und nach dem Trocken in ein Labor eingesandt. Dort werden an den Stellen, die mit einem Bluttropfen inkubiert worden sind, Scheiben von definierter Größe ausgestanzt und auf einen Nährboden aufgetragen, der mit den für die Untersuchung benötigten biologischen Materialien versehen ist.

Die bekannten Verfahren zeichnen sich vor allem dadurch aus, dass die Probenahme am Patienten vergleichsweise einfach erfolgen kann, nur eine sehr geringe Menge Blut benötigt wird und die Patientenprobe verhältnismäßig unkompliziert in ein Labor eingesendet werden kann.

Aus der EP 2 420 831 A1 ist eine Testkarte mit einem Testfeld bekannt, auf das Patientenproben in Form von Tropfen aufgebracht werden können. Wesentliches Merkmal der in dieser Druckschrift beschriebenen technischen Lösung ist, dass im Inneren der Testkarte eine Ausnehmung vorgesehen ist, in die ein Filterpapier zur Aufnahme der Bluttropfen eingespannt ist. Hierbei wird ein spezielles Filterpapier, nämlich sogenanntes Glasfaserpapier, verwendet, um ein Verlaufen der Blutprobe aus den hierfür vorgesehenen Bereichen in umliegende Bereiche zu vermeiden. Auf diese Weise soll eine genaue Analyse auch kleiner Moleküle ermöglicht werden.

Ausgehend von den bekannten Testkarten, den sogenannten Filterpapierkarten bzw. dryblotting Tests, bei denen im Labor vor einer Untersuchung der Blutprobe stets mit Hilfe eines Stanzgerätes der benötigte Probenbereich ausgestanzt werden muss, liegt der Erfindung die Aufgabe zu Grunde, eine Vorrichtung bzw. ein Verfahren derart weiterzubilden, dass die Probenahme und der Transport der Patientenprobe vergleichsweise einfach erfolgen kann und gleichzeitig die Verarbeitung der Probe im Labor vereinfacht wird. Insbesondere sollen hierbei die Handhabung der einzelnen Filterpapierkarten einerseits vereinfacht und zum anderen der Bedarf an zusätzlichen Laborgeräten minimiert werden. US5939259A ist Stand der Technik.

Die zuvor geschilderte Aufgabe wird mit einer Vorrichtung gemäß Anspruch 1 sowie einem Verfahren nach Anspruch 7 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Die Erfindung betrifft zunächst eine Vorrichtung zur Aufbewahrung und zum Transport einer Körperflüssigkeitsprobe gemäß Anspruch 1.

Das wesentliche Merkmal der erfindungsgemäßen technischen Lösung besteht somit darin, dass das Sorptionselement im Labor auf einfache Weise, insbesondere ohne Hilfswerkzeug, vom Tragelement und/oder dem Überlaufreservoir zu trennen ist. In Bezug auf die Erfindung wird unter manuell ohne Hilfswerkzeug verstanden, dass die Sollbruchstelle derart ausgeführt ist, dass das Sorptionselement händisch, also manuell ausschließlich per Hand vom Tragelement bzw. dem Überlaufreservoir abbrechbar ist. Wesentlich hierbei ist, dass keine zusätzliche Vorrichtung, insbesondere auch keine Stanzvorrichtung benötigt wird, um das Sorptionselement mit der Körperflüssigkeitsprobe von der Testvorrichtung mit Tragelement zu trennen.

Gemäß einer ersten speziellen Ausführungsform der Erfindung ist vorgesehen, dass sich die wenigstens eine Sollbruchstelle zwischen dem Überlaufreservoir und dem Sorptionselement befindet. Gemäß der Erfindung ist vorgesehen, dass an oder in dem als Grundkörper verwendeten Tragelement einer Testkarte ein Überlaufreservoir vorgesehen ist, das wiederum mit wenigstens einem Sorptionselement verbunden ist. Die Sollbruchstelle ist in diesem Fall auf vorteilhafte Weise zwischen dem Überlaufreservoir und dem Sorptionselement eingebracht. In einer bevorzugten Ausführungsform wird unter dem Begriff "Sollbruchstelle", wie hierin verwendet, eine Stelle verstanden, die so beschaffen ist, dass eine über die Längsachse gleichmäßige Verbiegung des gesamten Gegenstandes einen Bruch zuerst an dieser Stelle bewirkt. Beispielsweise kann die Sollbruchstelle durch Anritzen einer nicht permanent verformbaren, aber biegbaren Kunststoffschicht, bevorzugt einer Schicht aus einem Elastomer, erzeugt werden. Die Schicht wird dabei in einer Form angeritzt, die sich am Verlauf der erwünschten Bruchstelle orientiert, welche beim Bruch der Sollbruchstelle entsteht. In Abhängigkeit der benötigten Patientenproben und/oder der durchzuführenden Untersuchungen sind an oder in einer Testkarte wenigstens zwei, ganz besonders bevorzugt eine Mehrzahl von Sorptionselementen vorgesehen, wobei diese wiederum jeweils mit einem, besonders bevorzugt mit einem gemeinsamen Überlaufreservoir verbunden sind.

Wesentlich ist, dass die einzelnen Sorptionselemente über ein definiertes Füllvolumen verfügen, das sich danach bemisst, wie groß die Patientenprobe sein muss, damit eine qualitativ hochwertige Untersuchung der Probe erfolgen kann. In einer besonderen Ausführungsform der Erfindung wird bei der Dimensionierung eines Sorptionselements ferner berücksichtigt, dass sich dieses auf einfache Weise in eine becherförmige Ausnehmung einer Mikrotiterplatte, einem sogenannten Well, einfügen und/oder auf einen Blotstreifen aufbringen lässt. Der große Vorteil besteht in diesem Fall darin, dass sich die einzelnen Sorptionselemente nicht nur einfach vom Tragelement, insbesondere von einer Testkarte, manuell ohne Hilfswerkzeug abtrennen lassen, sondern dass sie darüber hinaus im Labor leicht weiter verarbeitbar sind. Sobald auf das Sorptionselement zu viel Körperflüssigkeit aufgebracht wird, also eine Menge, die über das definierte Füllvolumen hinausgeht, wird die überschüssige Menge der Patientenprobe automatisch vom Überlaufreservoir aufgesogen. In diesem Zusammenhang ist es gemäß einer speziellen Weiterbildung denkbar, dass das Überlaufreservoir das gleiche Material wie das Sorptionselement aufweist.

Gemäß einer weiteren besonderen Ausführungsform verfügt das Tragelement der erfindungsgemäß ausgeführten Vorrichtung über wenigstens ein Kennzeichenfeld, in das Informationen über Art und/oder Herkunft der Körperflüssigkeitsprobe und/oder über eine durchzuführende Untersuchung eintragbar sind. Auf vorteilhafte Weise werden auf dem als Grundkörper verwendeten Tragelement, insbesondere dem Grundkörper einer Testkarte, spezielle Felder vorgesehen, in die beispielsweise Angaben zu der untersuchten Person oder der gewünschten Untersuchung angegeben werden können.

In diesem Zusammenhang ist es denkbar, dass auf einer Seite einer Testkarte Angaben zum Patienten und auf der Seite Angaben zur durchzuführenden Untersuchung aufgeführt werden. Durch eine derartige räumliche Trennung der Angaben wird zuverlässig sichergestellt, dass Verwechslungen vermieden werden und die entsprechenden Karten im Labor vergleichsweise schnell ausgelesen werden können. Sofern Kennzeichnungen bzw. Kennzeichenfelder zur Wiedergabe von Informationen auf der Testkarte vorgesehen sind, können diese wahlweise als Schriftfelder und/oder als Felder, die sonstige Kennzeichnungen, vornehmlich Codes in Form von Barcodes, 3-D-Codes, Data-Matrix-Codes, enthalten, ausgeführt sein.

Selbstverständlich ist es ebenfalls denkbar, dass auf der Testkarte geeignete Felder vorgesehen sind, um beispielsweise mit Hilfe von Kreuzen oder anderen Kennzeichnungen gezielt Auswahlen, insbesondere hinsichtlich der gewünschten Untersuchungen, treffen zu können.

Bevorzugt umfasst das Tragelement wenigstens eine Schicht eines Elastomers, d. h. eines Kunststoffes, der durch Krafteinwirkung, insbesondere durch Biegen, kurzzeitig, aber nicht permanent bis zu einer gewissen Grenze reversibel verformt werden kann und danach, in Abwesenheit der Krafteinwirkung, wieder in seine ursprüngliche Form annimmt. Gemäß der Erfindung besteht das Trageelement aus einem Elastomer. Das Trageelement dient bevorzugt als Grundkörper für die Aufbringung sämtlicher Elemente der Testkarte, umfassend Sorptionselement und Überlaufreservoir.

Das Trageelement ist bevorzugt dichter als Papier, bevorzugt enthält es genug Kunststoff, am bevorzugtesten Elastomer, dass es nicht auf der Oberfläche einer wässrigen Reaktionslösung schwimmt, sondern mit beiden Seiten Kontakt zur wässrigen Phase ausbildet, spätestens nach kurzem Schütteln oder einmaligem Untertauchen. Dies erleichtert oder gestattet erst den Übergang von diagnostisch relevanten Stoffen aus einer auf dem Sorptionselement aufgebrachten Probe in die wässrige Phase.

Gemäß der Erfindung ist das Sorptionselement derart ausgeführt, dass es im Bereich von drei Außenflächen, die in unterschiedlichen Ebenen liegen, nicht an das Tragelement oder das Überlaufreservoir angrenzt, sondern diese Flächen frei nach außen stehen. In diesem Zusammenhang ist es denkbar, dass das Sorptionselement entweder in Form eines vorspringenden Elements nach außen über die eigentliche Testkarte hinaus steht oder aber innerhalb gezielt vorgesehener Ausnehmungen der Testkarte angeordnet ist. In jedem Fall ist das Sorptionselement aber derart angeordnet, dass es vergleichsweise einfach mit der Hand greifbar oder drückbar ist und so vom Tragelement und/oder dem Überlaufreservoir der Testkarte abbrechbar ist, insbesondere dadurch, dass es über ein die Sollbruchstelle aufweisendes Verbindungsstück mit dem Trageelement verbunden ist, wobei das Verbindungsstück bevorzugt eine Breite aufweist, die die Breite des Sorptionselements an seiner breitesten Stelle unterschreitet, bevorzugt um wenigstens 10, 20, 30 40, 50, 75 oder 90 % der breitesten Stelle des Sorptionselements. Bevorzugt ist das Sorptionselement dabei kreisförmig, d. h. die breiteste Stelle entspricht seinem Durchmesser. Die Verwendung von Werkzeugen, insbesondere einer Stanze, ist beim Abbrechen nicht erforderlich.

Gemäß der Erfindung weisen das Sorptionselement und das Überlaufreservoir ein Filterpapier auf. Bevorzugt wird für das Sorptionselement und/oder das Überlaufreservoir Filterpapier der Ahlstrom Filtration LLC. des Typs Ahlstrom 226 specimen collection paper mit der FDA-Zulassungsnummer K 062932 oder Whatman 903-Filterpapier der GE Healthcare mit der FDA-Zulassungsnummer K 932661. Der Durchmesser des Sorptionselements beträgt bevorzugt 4,5 bis 5,5 mm, insbesondere 5 mm. Das bevorzugte Füllvolumen liegt im Bereich von 3,3 bis 4,3 µl, insbesondere bei 3,5 µl.

In Ergänzung zur Verwendung eines Filterpapiers ist es denkbar, Fasermaterial für das Sorptionselement und/oder das Überlaufreservoir zu verwenden. Bei Verwendung eines speziellen Filtermaterials wir dieses derart ausgeführt, dass es das aufgetropfte Probenmaterial, insbesondere Blut, gut aufsaugt und sich beginnend vom Mittelpunkt des Sorptionselements eine gleichmäßig gesättigte Füllung einstellt, so dass sich das Probenmaterial im Labor auf einfache Weise und vor allem ohne erhebliche Materialverluste dem Sorptionselement entnehmen lässt. Als Fasermaterialien bieten sich bevorzugt Bi-Component Materialien aus Polyamid (besser als Nylon bekannt) und Polypropylen (PP) an. Die entsprechenden Fasergemische zeichnen sich vor allem durch ihre Saugeigenschaften und die oben beschriebene Eignung aus, einmal aufgesaugtes Material vergleichsweise verlustfrei wieder abgeben zu können. Gemäß einer ganz besonderen Ausführungsform der Erfindung hat das ein geeignetes Fasermaterial enthaltene Sorptionselement eine Stärke von 0,4 bis 1 mm, insbesondere von 0,45 mm, wobei das Fasermaterial über eine Dichte von 0,3 bis 0,4 g/cm³, insbesondere von 0,33 oder 0,37 g/cm³ verfügt.

Neben der beschriebenen Vorrichtung betrifft die Erfindung des Weiteren ein Verfahren gemäß Anspruch 7 zum Transport einer Körperflüssigkeitsprobe vom Ort einer Probenahme zum Ort einer Untersuchung, insbesondere zu einem Labor. Bei dem Verfahren wird während der Probenahme ein Tropfen der Körperflüssigkeit auf ein Sorptionselement, das an einem Tragelement einer Testvorrichtung, insbesondere in Form einer Karte, befestigt ist, aufgetropft. Eine über die für die Untersuchung benötigte definierte Menge der Körperflüssigkeit hinausgehende Überschussmenge wird hierbei automatisch von einem an das Sorptionselement angrenzenden Überlaufreservoir aufgesaugt. Daraufhin wird die Testvorrichtung zum Ort der Untersuchung, insbesondere einem Labor, verbracht und am Ort der Untersuchung das Sorptionselement mit der Körperflüssigkeitsprobe vom Tragelement der Testvorrichtung getrennt. Das erfindungsgemäße Verfahren zeichnet sich dadurch aus, dass das Sorptionselement händisch, also manuell ausschließlich per Hand, vom Tragelement und/oder vom Überschussreservoir abgebrochen wird. Die Verwendung eines zusätzlichen Werkzeugs, wie beispielsweise einer Stanze, ist bei Einsatz des erfindungsgemäßen Verfahrens nicht erforderlich.

Ein besonderer Vorteil der vorliegenden Erfindung besteht darin, dass das abgetrennte Sorptionselement, bevorzugt mit eine Blutprobe, noch bevorzugter einer getrockneten Blutprobe, anschließend direkt für Laboruntersuchungen eingesetzt werden kann. In einer bevorzugten Ausführungsform umfasst das erfindungsgemäße Verfahren daher weiterhin den Schritt Einbringen des abgetrennten Sorptionselement in eine wässrige Reaktionslösung, optional umfassend ein diagnostisch einsetzbares Antigen. Bei solchen Laboruntersuchungen kann es sich insbesondere um einen Immunoassay handeln, bei dem eine Probe in einem wässrigen Reaktionspuffer gelöst werden, wobei darin enthaltene Stoffe in die wässrige Phase übertreten. Bedeutend sind in diesem Zusammenhang insbesondere diagnostisch informative Antikörper, beispielsweise gegen infektiöse Agenzien wie pathogene Bakterien, Viren oder Parasiten, oder Antoantikörper von Bedeutung. Die so erhaltene wässrige Reaktionslösung kann zum Zwecke eines ELISA mit einem Antigen kontaktiert werden, dass bevorzugt an einer festen Phase immobilisiert ist, beispielsweise dem Boden eines Reaktionsgefässes, einem Träger oder einem Bead. Ein Antigen-(Auto)Antikörper-Komplex kann anschließend mit einem geeigneten Mittel nachgewiesen werden, z. B. mit einem sekundären Antikörper, welcher mit einem Label markiert ist, z.B. einem Enzym, einem radioaktiven Isotop, einer fluoreszenzfähigen chemischen Gruppe oder dergleichen. Auch eine Untersuchung der wässrigen Reaktionslösung mittels indirekter Immunfluoreszenz bzw. unter Kontaktierung eines Biochips ist möglich. Weitere diagnostische Verfahren neben Immunassays und Immunfluorezenz umfassen die diagnostische Nukleinsäure-Amplifikation mittels PCR. Geeignte Reagezien, darunter Antigene und sekundäre Antikörper, sind dem Fachmann bekannt und im Handel erhältlich.

Im Wege eines solchen Verfahrens erhältlich ist erfindungsgemäß ein Sorptionselement erhältlich durch Aufbringen einer flüssigen Probe, bevorzugt einer flüssigen Ganzblutprobe, auf ein Sorptionselement der Vorrichtung gemäß einem der Ansprüche 1 bis 6, gefolgt von den Schritten Abbrechen des Sorptionselements und optional Trocknen der Probe.

Erfindungsgemäß kann ein solches abgebrochenes Sorptionselement eingebracht werden in eine wässrige Reaktionslösung, die das erfindungsgemäße Sorptionselement umfasst, optional zusätzlich ein diagnostisch einsetzbares Antigen. Das Einbringen kann durch blosses Hineingeben, aber auch durch Untertauchen in der Reaktionslösung und/oder Schütteln darin vonstatten gehen. Die Bedingungen werden so gewählt, dass in der Probe enthaltene lösliche Stoffe in die wässrige Reaktionslösung übergehen können.

Erfindungsgemäß befindet sich eine derartige wässrige Reaktionslösung in einem Reaktionsgefäß, das zur Durchführung eines diagnostischen Verfahrens geeignet ist, bevorzugt in einer Mikrotiterplatte, die zahlreiche, bevorzugt mindestens oder mehr als 2, 4, 6, 8, 10, 12, 18, 24, oder 48 Wells geeignet zur parallelen Abarbeitung eben so vieler Proben parallel, umfasst. Die Größe und Form des Sorptionselementes kann an die Form der Wells angepasst sein.

Im Folgenden wird die Erfindung ohne Beschränkung des allgemeinen Erfindungsgedankens anhand von Ausführungsbeispielen unter Bezugnahme auf verschiedene Figuren beschrieben. Dabei zeigen:
- Figur 1:: Testkarte für einen Trockenbluttest (dry-Blot Test) mit nach außen abstehenden Sorptionselementen;
- Figur 2:: Testkarte für einen Trockenbluttest mit innen liegenden Sorptionselementen;
- Figur 3:: Testkarte mit ausstanzbarem Bereich, in dem innenliegende Sorptionselemente angeordnet sind und
- Figur 4:: Detailansicht des Überlaufreservoirs mit den daran befestigten Sorptionselementen.

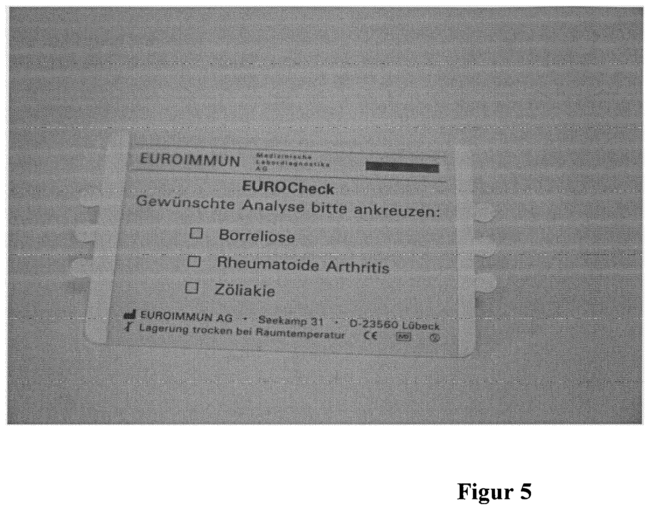

- Fig. 5:: Fotographische Darstellung einer besonders bevorzugten erfindungsgemäßen Ausführungsform.

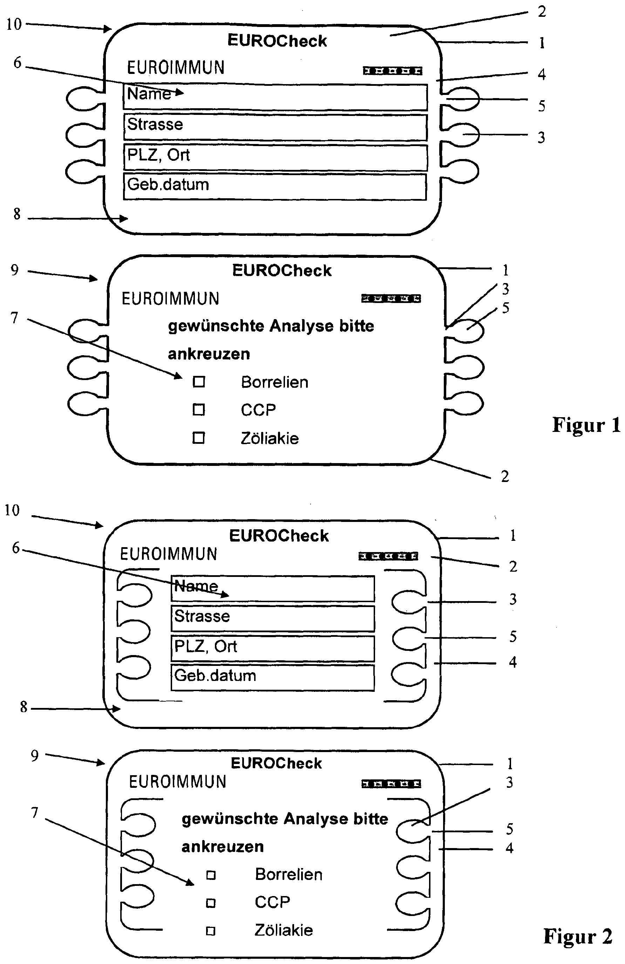

Figur 1 zeigt eine erfindungsgemäß ausgeführte Testkarte zum bevorzugten Transport von Blutproben eines Patienten in getrockneter Form vom Ort der Probenahme zu einem Labor. Die dargestellte Testkarte 1 verfügt im Wesentlichen über ein Tragelement 2, an dem beidseitig an den kurzen Seiten zum einen jeweils Überlaufreservoire 4 sowie jeweils drei Sorptionselemente 3 vorgesehen sind. Zwischen den Sorptionselementen 3, die beidseitig der Testkarte 1 vorgesehen sind, und den jeweiligen gemeinsamen Überlaufreservoiren 4 sind Sollbruchstellen 5 vorgesehen, so dass die Sorptionselemente 3 leicht durch abbrechen von der eigentlichen Testkarte 1 entfernt werden können. Ein Werkzeug ist hierfür nicht erforderlich. Auf diese Weise kann mit einfachen Mitteln die Entnahme einer Blutprobe beispielsweise in einer Arztpraxis, auf einer Klinikstation, in der Apotheke oder sogar zu Hause vorgenommen werden, indem ein Bluttropfen auf wenigstens eines der Sorptionselemente getropft wird, und die Karte schließlich zur Auswertung in ein Labor eingeschickt wird.

Zum Transport einer Testkarte 1 ist auf vorteilhafte Weise eine Testkartenhülle vorgesehen, in die die Testkarte 1 vollständig, insbesondere mit ihren Sorptionselementen 3, eingeschoben werden kann. Auf diese Weise wird sichergestellt, dass die Testkarte 1 verschickt und transportiert werden kann, ohne dass Beschädigungen, insbesondere an den Sorptionselementen 3 auftreten oder die Sorptionselemente sogar vorzeitig ungewollt abbrechen.

Bei den Sorptionselementen 3 handelt es sich um Faser- oder Filterpads mit einem definierten Volumen zur Aufnahme der jeweils zur Testauswertung benötigten Menge Blut. Angrenzend an die Sorptionselemente 3 ist beidseitig jeweils ein Überlaufreservoir 4, das das gleich Material aufweist wie die Sorptionselemente 3, vorgesehen, das automatisch überschüssiges Blut, welches vom Sorptionselement 3 nicht aufgenommen werden konnte, aufsaugt. Die Überlaufreservoire 4 sind hierbei jeweils ebenfalls beidseitig der Testkarte 1 an den kurzen Seiten unmittelbar am Tragelement 2 vorgesehen und stellen somit die Verbindung zwischen dem Tragelement 2 der Testkarte 1 und den einzelnen Sorptionselementen 3, in dem dargestellten Ausführungsbeispiel beidseitig jeweils drei, dar.

Sobald eine mit den entsprechenden Blutproben versehene Testkarte im Labor eingetroffen ist, können auf verhältnismäßig einfache Weise die einzelnen Sorptionselemente 3, die mit einer Blutprobe versehen sind, durch Abbrechen von der Testkarte 1 getrennt werden. Eine Stanze ist hierfür nicht erforderlich. Um darüber hinaus stets eine sichere Identifikation der Zugehörigkeit einer Blutprobe zu einem Patienten sicher zu stellen, verfügt die in Figur 1 dargestellte Testkarte 1 auf der einen Seite, der Testkartenrückseite 10, über insgesamt vier übereinander angeordnete Kennzeichenfelder 6, in die der Name, die Anschrift sowie das Geburtsdatum des Patienten, eingetragen werden kann. Weiterhin befindet sich im unteren Bereich ein Kennzeichenfeld 8, auf das das Verfallsdatum, die Chargenbezeichnung sowie eine Bestellnummer eingetragen werden.

Auf der dieser Seite gegenüberliegenden Vorderseite 9 der Testkarte 1 befindet sich ein weiteres Informationsfeld 7, mit drei übereinander angeordneten Kästchen, in denen ein Kreuz für die gewünschte Untersuchung eingetragen werden kann. Bei dem dargestellten Ausführungsbeispiel kann ausgewählt werden, ob eine Untersuchung in Bezug auf eine Erkrankung des Patienten mit Borreliose, Rheumatoider Arthritis und/oder Zöliakie durchgeführt werden soll.

Ferner ist im oberen Bereich der Vorderseite 9 der Testkarte 1 ausreichend Platz vorgesehen, um Produktbezeichnungen des Herstellers eintragen zu können, während im unteren Bereich weitere Informationen in Bezug auf den Hersteller selbst und beispielsweise die Lagerung der Testkarte 1 angegeben werden können.

Das Vorsehen definierter Flächen mit Filter- oder Faserpads als Sorptionselemente 3 und ein daran angrenzendes Überlaufreservoir 4 stellen sicher, dass eine einfache und bequeme Blutabnahme von sehr geringen Mengen Blut durchgeführt werden kann. Darüber hinaus werden ein einfacher Transport sowie eine definierte Dosierung einer Patientenprobe für die spätere Analyse im Labor sichergestellt. Die Filter- oder Faserpads sind jeweils durch ihre geometrische Form definiert und über eine Verbindung mit Sollbruchstelle 5 am Überlaufreservoir 4 befestigt. Aufgrund der Sollbruchstelle 5, die bevorzugt als Perforation oder Kerbe ausgeführt ist, kann das jeweils benötigte Sorptionselement 3 einfach und ohne Zuhilfenahme eines Werkzeugs vom Überlaufreservoir getrennt werden. Mit Hilfe der dargestellten Testkarte 1 ist somit auf bevorzugte Weise ein Patienten-Blutabnahme-Kit für den Arzt, die Apotheke oder den Patienten selbst bereit zu stellen, wobei die Testkarte 1 für den späteren Einsatz im Diagnostiklabor zuverlässig zu versenden ist.

Die einfache Handhabung der Testkarte 1 wird insbesondere dadurch sichergestellt, dass die Karte auf bevorzugte Weise über das sogenannte Checkkartenformat verfügt. Die in Figur 1 dargestellte Testkarte 1 hat eine Größe von 86 mm x 54 mm, wobei die beidseitig der kurzen Seiten vorgesehenen Überlaufreservoire 4 eine Breite von etwa 5 mm aufweisen. Die einzelnen Sorptionselemente 3 sind in einem Abstand von etwa 4 mm zueinander angeordnet und weisen parallel zur Endkante der Testkarte 1 eine Breite von ca. 5 mm auf.

In Ergänzung zu Figur 1 zeigt Figur 2 eine erfindungsgemäß ausgeführte Testkarte 1, bei der die Sorptionselemente 3 nicht in Form von nach außen ragenden Vorsprüngen ausgeführt sind, sondern nach innen in eine größere Ausnehmung der Testkarte 1 hineinragen. Wiederum verfügt die Testkarte 1 über ein Tragelement 2, an dem jeweils zwei Überlaufreservoire 4 mit wiederum jeweils drei Sorptionselementen 3 befestigt sind. Wesentlich hierbei ist, dass zwischen den als Filter- oder Faserpads ausgeführten Sorptionselementen 3 und den Überlaufreservoiren 4 Sollbruchstellen 5 vorgesehen sind. Diese Sollbruchstellen 5 in Form einer Perforation oder Kerbe ermöglichen ein einfaches Abbrechen bzw. Ausbrechen der Sorptionselemente 3 aus der Testkarte 1. Ferner verfügt die Testkarte 1 wiederum auf ihrer Vorderseite 9 über ein Kennzeichenfeld 7, das die Auswahl der gewünschten Untersuchung ermöglicht, während auf der Rückseite 10 Kennzeichenfelder 6 vorgesehen sind, die eine Identifikation des Patienten ermöglichen.

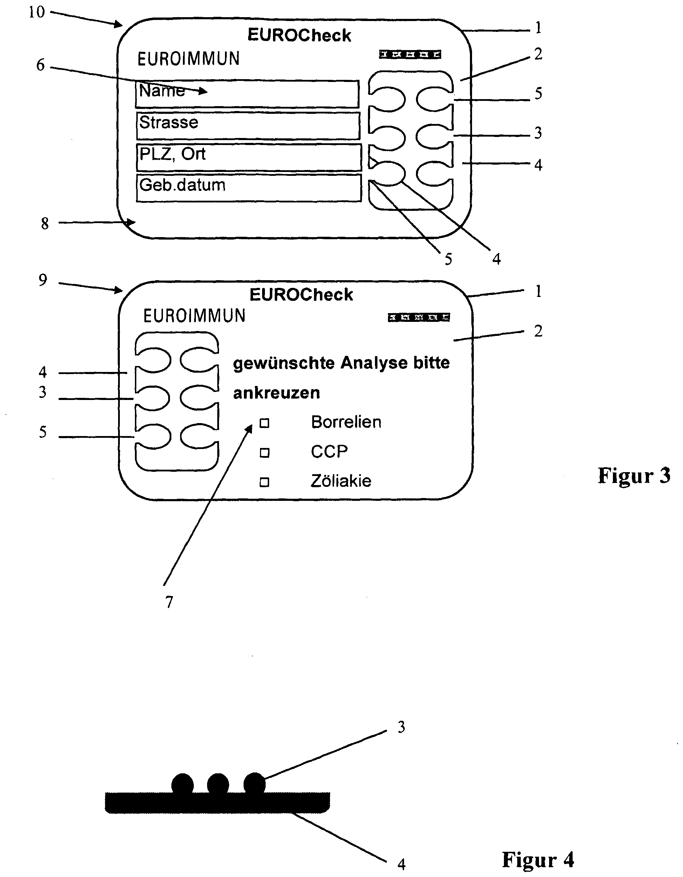

Figur 3 zeigt eine im Vergleich zu den bisher erläuterten technischen Lösungen alternative Ausführungsform einer erfindungsgemäßen Testkarte 1. Die in Figur 3 gezeigte Testkarte 1 verfügt wiederum über eine Vorder- und einer Rückseite 9, 10, wobei auf der Vorderseite 9 die gewünschte Untersuchung angekreuzt und auf der Rückseite 10 Name, Anschrift und Geburtsdatum des Patienten eingetragen werden können.

In diesem Fall ist lediglich ein Überlaufreservoir 4 auf der Testkarte 1 vorgesehen, das mit insgesamt sechs Sorptionselementen 3 in Verbindung steht. Überschüssiges Probenmaterial, das nicht mehr im Füllvolumen des jeweiligen Sorptionselements 3 aufgenommen werden kann, wird wiederum vom Überlaufreservoir 4 adsorbiert.
Zwischen den einzelnen Sorptionselementen 3 und dem Überlaufreservoir 4 befinden sich auch in diesem Fall Sollbruchstellen 5. Auf diese Weise wird sichergestellt, dass die einzelnen Sorptionselemente 3 im Labor durch einfaches Herausbrechen ohne Werkzeug aus der Testkarte 1, insbesondere vom Überlaufreservoir 4 und dem Tragelement 2 entfernt werden können.

Figur 4 zeigt in einer schematisierten Detaildarstellung die Anordnung eines Überlaufreservoirs 4 mit daran befestigten Sorptionselementen 3. Hierbei ist es grundsätzlich unerheblich, ob es sich bei den Sorptionselementen 3 um in Bezug auf die Testkarte 1 außen liegende oder innen liegende Sorptionselemente 3 handelt. Wesentlich ist, dass das Überlaufreservoir 4 aus dem gleichen Material wie die als Sorptionselemente 3 vorgesehenen Filter- oder Faserpads ausgeführt ist, also insbesondere das gleiche Material verwendet wird.

Das Überlaufreservoir 4 ist derart ausgeführt und mit den Sorptionselementen 3 verbunden, dass während der Einbringung einer Blutprobe in ein Sorptionselement 3 vom Überlaufreservoir 4 automatisch überschüssiges Blut, also die Blutmenge einer Probe, die die definierte Füllmenge eines Filter- oder Faserpads übersteigt, aufgesaugt wird. Zwischen den Sorptionselementen 3 und dem Überlaufreservoir 4 sind wiederum geeignete Sollbruchstellen 5 vorgesehen. Derartige Sollbruchstellen 5 werden durch einzelne Lochungen bzw. eine Perforation oder auch Einkerbungen im Verbindungsbereich zwischen den Sorptionselementen 3 und dem Überlaufreservoir 4 hergestellt. Diese Maßnahme stellt sicher, dass die einzelnen Sorptionselemente 3 ohne Werkzeug vom Überlaufreservoir 4 abgebrochen werden können. Ausdrücklich wird darauf hingewiesen, dass selbstverständlich nur jene Sorptionselemente 3 von dem jeweiligen Überlaufreservoir 4 bzw. der Testkarte 1 entfernt werden, die eine zu untersuchende Blutprobe aufweisen.

### Bezugszeichenliste

- 1: Testkarte
- 2: Tragelement
- 3: Sorptionselement
- 4: Überlaufreservoir
- 5: Sollbruchstelle
- 6: Kennzeichenfeld für probenspezifische Daten
- 7: Kennzeichenfeld für untersuchungsspezifische Daten
- 8: Kennzeichenfeld für testkartenspezifische Daten
- 9: Vorderseite der Testkarte
- 10: Rückseite der Testkarte
- 11: Verbindungsstück

## Patentansprüche

1. Vorrichtung zur Aufbewahrung und zum Transport einer Körperflüssigkeitsprobe mit einem einen Grundkörper bildenden Tragelement (2), an dem wenigstens ein Sorptionselement (3), das ein definiertes Füllvolumen aufweist und geeignet ist, Körperflüssigkeit aufzunehmen, und ein an das Sorptionselement (3) angrenzendes Überlaufreservoir (4), das Körperflüssigkeit aus dem Sorptionselement (3) aufnimmt sobald das Füllvolumen des Sorptionselementes (3) gefüllt ist, befestigt sind,
**dadurch gekennzeichnet, dass** zumindest eine Sollbruchstelle (5) vorgesehen ist, die derart ausgeführt ist, dass das Sorptionselement (3) manuell ohne Hilfswerkzeug vom Tragelement (1) und/oder vom Überlaufreservoir trennbar ist,
wobei das Überlaufreservoir (4) und das Sorptionselement (3) ein Filterpapier aufweist;
wobei das Tragelement (2) aus einem Elastomer besteht;
wobei das Sorptionselement (3) wenigstens drei gegenüber einer Umgebung frei liegende und in unterschiedlichen Ebenen angeordnete Flächenbereiche aufweist; und
wobei das Überlaufreservoir (4) derart ausgeführt, dass dieses Körperflüssigkeit aus dem Sorptionselement (3) aufnimmt sobald das Füllvolumen des Sorptionselements (3) vollständig mit Körperflüssigkeit gefüllt ist.

2. Vorrichtung nach Anspruch eins, **dadurch gekennzeichnet dass** das Sorptionselement (3) vom Tragelement (2) und/oder vom Überlaufreservoir händisch, also manuell ausschließlich per Hand, abbrechbar ist.

3. Vorrichtung nach Anspruch 1 oder 2, **dadurch gekennzeichnet, dass** sich die wenigstens eine Sollbruchstelle (5) zwischen dem Überlaufreservoir (4) und dem Sorptionselement (3) befindet.

4. Vorrichtung nach einem der Ansprüche 1 bis 3, **dadurch gekennzeichnet, dass** das Tragelement (2) ein Kennzeichenfeld (6, 7) aufweist, in das Informationen über Art und/oder Herkunft der Körperflüssigkeitsprobe und/oder über eine durchzuführende Untersuchung eintragbar sind.

5. Vorrichtung nach Anspruch 4, **dadurch gekennzeichnet dass** das Tragelement (2) auf einer ersten Seite ein Kennzeichenfeld (6) aufweist, in das Informationen über Art und/oder Herkunft der Körperflüssigkeitsprobe eintragbar sind, und auf einer zweiten, der ersten Seite gegenüberliegenden Seite über ein Kennzeichenfeld (7) verfügt, in das Angaben zur durchzuführenden Untersuchung eintragbar sind.

6. Vorrichtung nach einem der Ansprüche 1 bis 5, **dadurch gekennzeichnet, dass** das Sorptionselement (3) und/oder das Überlaufreservoir (4) wenigstens ein Fasern enthaltendes Material aufweist.

7. Verfahren zum Transport einer Körperflüssigkeitsprobe vom Ort einer Probenahme zum Ort einer Untersuchung, insbesondere zu einem Labor, bei dem bei der Probenahme Körperflüssigkeit auf ein Sorptionselement (3), das an einem als Grundkörper einer Testvorrichtung (1) dienenden Tragelement (2) befestigt ist, aufgetropft wird, wobei eine über die für die Untersuchung benötigte definierte Menge der Körperflüssigkeit hinausgehende Überschussmenge automatisch von einem an das Sorptionselement (3) angrenzenden Überlaufreservoir (4) aufgesaugt wird, bei dem die Testvorrichtung (1) zum Ort der Untersuchung verbracht wird und bei dem am Ort der Untersuchung das Sorptionselement (3) mit der Körperflüssigkeitsprobe vom Tragelement (2) der Testvorrichtung (1) getrennt wird,
**dadurch gekennzeichnet dass** das Sorptionselement (3) händisch, also manuell ausschließlich per Hand, vom Tragelement (2) und/oder vom Überlaufreservoir (4) abgebrochen wird,
wobei das Überlaufreservoir (4) und das Sorptionselement (3) ein Filterpapier aufweist;
wobei das Tragelement (2) aus einem Elastomer besteht;
wobei das Sorptionselement (3) wenigstens drei gegenüber einer Umgebung frei liegende und in unterschiedlichen Ebenen angeordnete Flächenbereiche aufweist; und
wobei das Überlaufreservoir (4) derart ausgeführt, dass dieses Körperflüssigkeit aus dem Sorptionselement (3) aufnimmt sobald das Füllvolumen des Sorptionselements (3) vollständig mit Körperflüssigkeit gefüllt ist.

8. Verfahren nach Anspruch 7, weiterhin umfassend den Schritt Einbringen des abgetrennten Sorptionselements in eine wässrige Reaktionslösung, optional umfassend ein diagnostisch einsetzbares Antigen.

9. Verfahren nach Anspruch 7 oder 8, **dadurch gekennzeichnet, dass** eine flüssige Probe, insbesondere eine flüssige Ganzblutprobe, auf das Sorptionselement (3) aufgebracht wird.

10. Verfahren nach einem der Ansprüche 7 bis 9, **dadurch gekennzeichnet, dass** die auf das Sorptionselement aufgebrachte Probe nach dem Abbrechen des Sorptionselements (3) vom Tragelement (2) und/oder vom Überlaufreservoir (4) getrocknet wird.

11. Verfahren nach einem der Ansprüche 7 bis 10, **dadurch gekennzeichnet, dass** das abgebrochene Sorptionselement (3) mit einer wässrigen Reaktionslösung, insbesondere mit einer wässrigen Reaktionslösung, die ein diagnostisch einsetzbares Antigen umfasst, in Kontakt gebracht wird.

12. Verfahren nach Anspruch 11, **dadurch gekennzeichnet, dass** das abgebrochene Sorptionselement (3) in ein Reaktionsgefäß, insbesondere eine Multititerplatte, eingebracht wird, die die wässrige Reaktionslösung umfasst.

## Claims

1. Device for storage and transport of a body fluid sample with a supporting element forming a base body (2), attached on which is at least one sorption element (3), which has a defined filling volume and is suitable to absorb body fluid, and an overflow reservoir adjacent to the sorption element (3), which absorbs body fluid from the sorption element (3) as soon as the filling volume of the sorption element is filled (3), are attached,
**characterized in that** at least one predetermined breaking point (5) is provided, which is designed in such a way that the sorption element (3) is manually separable from the support element (1) and/or from the overflow reservoir without an auxiliary tool,
wherein the overflow reservoir (4) and the sorption element (3) include a filter paper;
wherein the supporting element (2) consists of an elastomer;
wherein the sorption element (3) has at least three surface areas exposed to a surrounding area and arranged in different planes; and
wherein the overflow reservoir (4) is designed in such a way that it absorbs body fluid from the sorption element (3) as soon as the filling volume of the sorption element (3) is completely filled with body fluid.

2. Device according to claim one, **characterized in that** the sorption element (3) is manually separable from the support element (2) and/or from the overflow reservoir, i.e. manually limited to the hand.

3. Device according to claim 1 or 2, **characterized in that** the at least one predetermined breaking point (5) is located between the overflow reservoir (4) and the sorption element (3).

4. Device according to any one of claims 1 to 3, **characterized in that** the supporting element (2) has a label field (6, 7) into which information on the type and/or origin of the body fluid sample and/or about an examination to be carried out are recordable.

5. Device according to claim 4, **characterized in that** the supporting element (2) has a label field on a first side (6) into which information on the type and/or origin of the body fluid sample are recordable, and has on a second side opposing the first side a label field (7) into which information about an examination to be carried out are recordable.

6. Device according to any one of claims 1 to 5, **characterized in that** the sorption element (3) and/or the overflow reservoir (4) has at least one fiber-containing material.

7. Method for transporting a body fluid sample from the place of sampling to the place of examination, in particular to a laboratory in which, during sampling, body fluid is dripped on a sorption element (3), which is attached to a supporting element (2) serving as the base body of a test device (1), wherein an amount in excess of the defined amount of the body fluid required for the examination is automatically absorbed by an overflow reservoir adjacent to the sorption element (3), wherein the test device (1) is moved to the place of examination and wherein, at the place of examination, the sorption element (3) with the body fluid sample is separated from the supporting element (2) of the test device (1),
**characterized in that** the sorption element (3) is broken off manually from the support element (2) and/or from the overflow reservoir (4), i.e. manually limited to the hand,
wherein the overflow reservoir (4) and the sorption element (3) include a filter paper;
wherein the supporting element (2) consists of an elastomer;
wherein the sorption element (3) has at least three surface areas exposed to a surrounding area and arranged in different planes; and
wherein the overflow reservoir (4) is designed in such a way that it absorbs body fluid from the sorption element (3) as soon as the filling volume of the sorption element (3) is completely filled with body fluid.

8. The method according to claim 7, further comprising the step of placing the separated sorption element into an aqueous reaction solution, optionally comprising a diagnostically acceptable antigen.

9. The method according to claim 7 or 8, **characterized in that** a liquid sample, in particular a liquid whole blood sample, is applied to the sorption element (3).

10. The method according to any one of claims 7 to 9, **characterized in that** the sample applied to the sorption element is dried after breaking off the sorption element (3) from the support element (2) and/or from the overflow reservoir (4).

11. The method according to any one of claims 7 to 10, **characterized in that** the broken off sorption element (3) is brought into contact with an aqueous reaction solution, in particular with an aqueous reaction solution, which comprises a diagnostically acceptable antigen.

12. The method according to claim 11, **characterized in that** the broken off sorption element (3) is inserted into a reaction vessel, in particular a multititer plate, which comprises the aqueous reaction solution.

## Revendications

1. Dispositif permettant de conserver et de transporter un échantillon de fluide corporel, comprenant un élément support (2) formant un corps de base et auquel sont fixés au moins un élément de sorption (3) présentant un volume de remplissage défini et adapté pour recevoir un fluide corporel, et un réservoir de trop-plein (4) adjacent à l'élément de sorption (3) qui reçoit un fluide corporel de l'élément de sorption (3) dès que le volume de remplissage de l'élément de sorption (3) est rempli,
**caractérisé en ce qu'**au moins un point de rupture (5) est prévu qui est réalisé de telle sorte que l'élément de sorption (3) peut être séparé manuellement de l'élément support (1) et/ou du réservoir de trop-plein sans instrument auxiliaire,
le réservoir de trop-plein (4) et l'élément de sorption (3) comportant un papier-filtre ;
l'élément support (2) étant composé d'un élastomère ;
l'élément de sorption (3) présentant au moins trois zones de surface exposées à un environnement et disposées dans différents plans ; et
le réservoir de trop-plein (4) étant réalisé de telle sorte qu'il absorbe un fluide corporel provenant de l'élément de sorption (3) dès que le volume de remplissage de l'élément de sorption (3) est complètement rempli de fluide corporel.

2. Dispositif selon la revendication 1, **caractérisé en ce que** l'élément de sorption (3) peut être sectionné à la main de l'élément support (2) et/ou du réservoir de trop-plein, donc manuellement, exclusivement avec les mains.

3. Dispositif selon la revendication 1 ou 2, **caractérisé en ce que** ledit au moins un point de rupture (5) se trouve entre le réservoir de trop-plein (4) et l'élément de sorption (3).

4. Dispositif selon l'une quelconque des revendications 1 à 3, **caractérisé en ce que** l'élément support (2) présente un champ d'identification (6, 7) dans lequel on peut entrer des informations concernant le type et/ou l'origine de l'échantillon de fluide corporel et/ou un examen à effectuer.

5. Dispositif selon la revendication 4, **caractérisé en ce que** l'élément support (2) présente sur un premier côté un champ d'identification (6) dans lequel on peut entrer des informations concernant le type et/ou l'origine de l'échantillon de fluide corporel, et dispose sur un deuxième côté opposé au premier côté d'un champ d'identification (7) dans lequel on peut entrer des renseignements concernant l'examen à pratiquer.

6. Dispositif selon l'une quelconque des revendications 1 à 5, **caractérisé en ce que** l'élément de sorption (3) et/ou le réservoir de trop-plein (4) présentent au moins un matériau contenant des fibres.

7. Procédé de transport d'un échantillon de fluide corporel du lieu de prélèvement d'échantillon jusqu'au lieu d'un examen, en particulier un laboratoire, dans lequel, lors du prélèvement d'échantillon, un fluide corporel est appliqué par gouttes sur un élément de sorption (3) fixé à un élément support (2) servant de corps de base d'un dispositif de test (1), dans lequel une quantité excessive dépassant la quantité définie du fluide corporel nécessaire à l'examen est absorbée automatiquement par un réservoir de trop-plein (4) adjacent à l'élément de sorption (3), dans lequel le dispositif de test (1) est amené sur le lieu de l'examen, et sur le lieu de l'examen, l'élément de sorption (3) avec l'échantillon de fluide corporel est séparé de l'élément support (2) du dispositif de test (1),
**caractérisé en ce que** l'élément de sorption (3) est sectionné à la main de l'élément support (2) ou du réservoir de trop-plein (4), donc manuellement, exclusivement avec les mains,
le réservoir de trop-plein (4) et l'élément de sorption (3) présentant un papier-filtre ;
l'élément support (2) étant composé d'un élastomère ;
l'élément de sorption (3) présentant au moins trois zones de surface exposées à un environnement et disposées dans différents plans ; et
le réservoir de trop-plein (4) étant réalisé de telle sorte qu'il absorbe un fluide corporel provenant de l'élément de sorption (3) dès que le volume de remplissage de l'élément de sorption (3) est complètement rempli de fluide corporel.

8. Procédé selon la revendication 7, comprenant en outre l'étape consistant à introduire l'élément de sorption séparé dans une solution aqueuse de réaction, comprenant en option un antigène utilisable pour le diagnostic.

9. Procédé selon la revendication 7 ou 8, **caractérisé en ce qu'**un échantillon liquide, en particulier un échantillon liquide de sang complet, est appliqué à l'élément de sorption (3).

10. Procédé selon l'une quelconque des revendications 7 à 9, **caractérisé en ce que** l'échantillon appliqué à l'élément de sorption est séché après que l'élément de sorption (3) a été sectionné de l'élément support (2) et/ou du réservoir de trop-plein (4).

11. Procédé selon l'une quelconque des revendications 7 à 10, **caractérisé en ce que** l'élément de sorption sectionné (3) est mis en contact avec une solution aqueuse de réaction, en particulier avec une solution aqueuse de réaction comprenant un antigène utilisable pour le diagnostic.

12. Procédé selon la revendication 11, **caractérisé en ce que** l'élément de sorption sectionné (3) est introduit dans une cuve de réaction, en particulier une plaque de multi-titrage, qui comprend la solution aqueuse de réaction.
